# EUROPEAN PATENT SPECIFICATION

(11) **EP 2 677 982 B1**
(45) Date of publication and mention of the grant of the patent: **17.08.2016**
(21) Application number: 12714036.6
(22) Date of filing: 24.02.2012
(51) Int. Cl.: A61F 9/08, G09B 21/00

(54) **AN OPTICAL DEVICE FOR THE VISUALLY IMPAIRED**
OPTISCHE VORRICHTUNG FÜR SEHBEHINDERTE
DISPOSITIF OPTIQUE POUR MALVOYANT

(30) Priority: 24.02.2011 GB 201103200
(43) Date of publication of application: 01.01.2014
(73) Proprietor: Oxford University Innovation Limited, Oxford OX2 0SZ (GB)
(72) Inventor: HICKS, Stephen, Oxford Oxfordshire OX3 9DU (GB)
(74) Representative: Thurston, Mark
(86) International application number: PCT/GB2012/050428
(87) International publication number: WO 2012/114123

(56) References cited:
- US-A- 5 060 062
- US-A1- 2006 011 718

## Description

The present invention relates to apparatus for aiding visual impairment and particularly relates to an optical device for visually-impaired individuals and to a method of operating such an optical device. There are around 370,000 individuals in the UK who are registered blind or partially sighted, and there are many more who suffer from some form of visual impairment or sight impediment that hinders their mobility or otherwise lessens their quality of life. However, for the majority of visually-impaired individuals at least some residual visual function remains, even for those who are registered as blind. This "residual visual function" may often be limited to the ability to simply discriminate between light and dark, but can also occasionally allow different colours to be distinguished from each other. Hence, for instance, many visually-impaired individuals are able to "see" a moving hand, but cannot count the separate fingers etc.

The loss of sight obviously impacts greatly on an individual's ability to navigate and negotiate their environment, and thus many individuals suffer reduced mobility as a result of their visual impairment. Statistics collated by the Royal National Institute of Blind People (RNIB) in the UK show that around 48 percent of blind or partially sighted individuals feel 'moderately' or 'completely' cut off from society. Typically, the only mobility aids available to visually-impaired individuals (notwithstanding guide dogs) are manual probes, namely the cane (i.e. white stick) or auditory devices (similar to echo locating equipment). However, our sense of sight is the most natural sense by which an individual becomes aware of their spatial environment and therefore even with the conventionally available aids, an individual is still likely to suffer from a reduced awareness of their environment, which diminishes their ability to safely navigate and negotiate obstacles in their immediate vicinity.

To some extent the prior art has attempted to address the issue of reduced mobility for visually-impaired individuals by providing various head-mounted augmented-reality devices. Said devices are for example described in the following documents: US 5,060,062 or WO94/16424.

However, most of these devices employ techniques for providing an 'enhanced' image to the individual, such that a camera captures an image of the individual's environment and processes that image to increase the brightness and contrast in the image. In addition, edge delineating and/or sharpening algorithms may also be applied which delineate edges in the image for the individual, thereby potentially improving their ability to discriminate between different types of object. Although such devices can improve the quality of life for an individual they are not universally effective for all visually-impaired sufferers, as a reasonable degree of actual vision is still required to view the images, which necessarily requires that the individual actually focus on a presented image to resolve information contained therein. Hence, for severely sighted individuals the ability to focus on an image may not be possible and therefore no degree of image enhancement can assist their mobility within their environment.

In addition, many of the known head-mounted devices are quite bulky and reasonably heavy in weight, so prolonged use of a augmented-reality headset may cause discomfort to the head and neck of a wearer, which could be particularly problematic for elderly wearers etc. Moreover, such headsets may not be aesthetically pleasing and so can cause an individual to feel 'self-conscious' about their condition as the headset may bring undue attention to them.

Therefore, it is an object of the present invention to address some, if not all, of the above problems in the art, by providing a device for aiding visually-impaired individuals which allows an individual to make use of at least some of their residual visual function to gain awareness of their spatial environment.

It is a further object of the present invention to provide a relatively lightweight visual aid for improving comfort and wearability for a visually-impaired individual.

According to a first aspect of the present disclosure there is provided an optical device for a visually-impaired individual, comprising
a spaced array of discrete light sources;
a support arranged to maintain the array in proximate relation to at least one eye of the individual; and
an image capture device configured to capture images of at least part of the individual's immediate environment;
wherein the array is configured to convey information to the individual by selectively illuminating one or more of the discrete light sources based on the content of the captured images.

By "visually-impaired individual" we mean an individual of any age or gender who has a visual impairment to their sight that reduces, diminishes or otherwise impedes their vision below that of an average-sighted person. In particular, the phrase is intended to include, but not be limited to, individuals who are registered as blind or partially-sighted, but in any event retain at least some residual visual function that permits some degree of discrimination between light and dark, and possibly also colour. Moreover, it is to be understood that no limitation is to be implied as to the cause of the visual impairment, and therefore the sight may be impeded by any hereditary or congenital condition, through age or as a result of injury etc.

The provision of an optical device which comprises a spaced array of discrete light sources in order to convey information to a visually-impaired individual by selectively illuminating one or more of the discrete light sources based on the content of a captured image of the individual's immediate environment is found to be particularly advantageous, as the individual is able make use of their residual visual function in order to gain at least a spatial awareness of their surroundings.

In this way, by virtue of the selective illumination of the light sources, information relevant to objects, and the distances to those objects, in the individual's environment can be conveyed to the individual to thereby enable the individual to navigate and negotiate their environment. As a result, safety for the individual is consequently significantly improved, as the individual has a better spatial knowledge of their surroundings which greatly improves their mobility within that environment and reduces risk of accident or injury.

The spaced array of discrete light sources is preferably in the form of a regular matrix of individual light sources that are spaced from one another by a predetermined amount. In preferred embodiments, the spaced array comprises a matrix of light emitting diodes (LEDs), each diode preferably being individually addressable so that each diode can be separately controlled. An advantage of using LEDs is that these require relatively lower levels of electrical power than other forms of light source, and are generally relatively lightweight and robust components.

The matrix of LEDs may be comprised of pure white LEDs, or alternatively, multi-colour LEDs for example, single diode dual-colour red and green LEDs or separate red and green LEDs etc. Of course, it is to be appreciated that any number or combination of white, coloured or multi-coloured LEDs (single or dual/multi-colour) may be used in the array of the present invention depending on the particular application or visual impairment of the individual.

An advantage of using differently coloured LEDs is that additional and/or more specific information may be conveyed to those individuals who possess some degree of colour discrimination. Therefore, as opposed to simply discriminating between light and dark, certain colours or combinations of colours can be assigned particular meanings, which may be used to convey different types of information or instructions to the wearer of the optical device.

However, where the individual has no residual colour perception, the required information may still be conveyed to the wearer by way of a white light, without any loss of spatial awareness or information. In such arrangements, other techniques of driving the LEDs (e.g. via spatial and/or temporal patterns) may be used, as will be discussed later. Indeed, in some preferred embodiments, the spaced array comprises a matrix of pure white light LEDs.

The support is arranged to maintain the array in proximate relation to at least one eye of the wearer of the optical device. Preferably, the support is configured such that it is able to hold the array at a distance from the eye which is substantially closer than the minimum focal length of the eye (i.e. the shortest distance at which focus can theoretically be attained). In other words, the array may be located at a distance from the wearer's eye such that the wearer does not need to focus on the light sources in the array. The array therefore preferably resides between around 3 to 5 cm from the wearer's eye in most cases. However, the exact distance will depend on the particular individual and their visual-impairment, so it is possible that the array may need to be spaced further from the eye, or closer to the eye, in some applications.

An advantage of placing the array close to the eye is that the intensity of the light received on the wearer's eye can be increased, which potentially enhances the perception between light and dark. Moreover, as there is no need to focus on the array, the optical device may be used by visually-impaired individuals who have little or no focussing ability, contrary to the augmented-reality headsets of the prior art which require the wearer to focus on an enhanced image.

In particularly preferred embodiments, the optical device further comprises a second spaced array of discrete light sources, such that each array is configured to convey information to a respective eye of the wearer. The second array is preferably structurally and functionally the same as the first array. However, in some embodiments the arrays could differ from each other depending on the particular application and/or visually-impaired individual (e.g. the wearer has colour perception in one eye only etc.).

The support most preferably comprises a spectacle frame. The frame may have foldable arms or alternatively the arms may be fixedly attached to the remaining portion (i.e. lens holder) of the frame. In addition or alternatively, the spectacle frame may be of a 'wrap around' type, so as to make better use of any peripheral vision and/or improve comfort or convenience for the individual. An advantage of using a spectacle frame for the support is that no relatively heavy head-mounted structural components are required, which reduces the overall weight of the optical device and thereby improves comfort for the wearer. Moreover, the use of a spectacle frame arguably improves the aesthetic appearance of the optical device, which may allow the wearer to feel more 'comfortable' when using the device in public as it is more discrete than a bulky headset.

Of course, it is to be appreciated that any other form of suitable lightweight support may be used with the optical device of the present invention, and therefore a spectacle frame is not intended to be limiting. In particular, by way of example, a 'flip-down' visor arrangement could alternatively be used that is clipped onto a headband or the brim of a hat or cap etc.

Where the support is in the form of a spectacle frame, each spaced array is preferably integrated into a respective 'display' in the shape or form of a spectacle lens fitted into each lens socket of the frame. The lenses themselves are preferably merely supports, holders or substrates for the matrix of LEDs and consequently preferably provide no optical correction to the wearer's vision. Hence, in preferred embodiments the lenses are made from a plastic material, which may be either transparent or opaque depending on the particular application and/or wearer.

In some embodiments, the LEDs can therefore be mounted onto the front or rear surfaces of the lens, or both, via adhesive etc. or alternatively can be integrally moulded (together with their electrical connections) into the material of the lens. In another embodiment, the LEDs may be mounted onto a transparent conductive film which may then be applied to the surface of the lens.

Of course, it is to be appreciated that any suitable technique or process for integrating, coupling or otherwise attaching the arrays to the lenses may be used in conjunction with the present invention depending on the particular application.

The dimensions of the arrays are preferably equivalent to a typical spectacle lens and preferably extend across the lens from top to bottom and from side to side. Hence, in particularly preferred embodiments the arrays may be approximately 35 x 30 mm and most preferably comprise at least 48 individually addressable LEDs (e.g. of a sort measuring approximately 2 x 1 mm each) in a preferably landscape hexagonal configuration.

However, it is to be appreciated that any number of LEDs and any appropriate configuration may be used in conjunction with the present invention. Indeed, as noted previously, the configuration of the arrays may differ between each eye, so that different types of information can be conveyed to the wearer depending on their particular visual impairment or individual eye function.

However, it is envisaged that the configuration of the arrays will likely be the same for all types of blindness, allowing one universal device to be used for all, but the arrays will be driven differently for specific wearers and/or certain types of visual-impairment and/or conditions etc. Hence, for example, colour can be disabled for individuals having no residual colour perception, while a reduced number (i.e. subset) of distributed LEDs (e.g. widely spaced, such as at the edges of the lens) can be driven for conditions where a wearer has difficulty distinguishing between different sources of light (e.g. where light/colour blurring is a problem).

The image capture device is most preferably mounted on the support itself, which in the examples of the spectacle frame, enables the image capture device to be integrated into the frame or else attached accordingly.

Preferably, the image capture device comprises at least one wide-angle camera, which is most preferably a miniature video camera of the CMOS or CCD type, for example. By "wide-angle camera" we mean a camera comprising an imaging lens that is able to image a scene that preferably subtends a large angle of between around 60-120 degrees or more etc. The camera is most preferably a colour video camera.

In particularly preferred embodiments, the image capture device comprises two wide-angle cameras, with each preferably being located at a respective upper edge corner of the spectacle frame, substantially above each display lens/array. An advantage of using two image capture devices relatively spaced from one another is that stereoscopic images of the wearer's immediate environment can be captured, which permits distance information to be determined for objects and obstacles etc. surrounding the wearer (as will be discussed later).

Another advantage of mounting the cameras on the spectacle frame above the lenses is that the captured images track or follow the wearer's line of sight, so that when the wearer turns his/her head the cameras image whatever is located along that particular direction. In this way, the wearer can build up a mental picture of his/her immediate environment by virtue of the information conveyed to the wearer via the LED arrays.

It should be appreciated, however, that the image capture device could be mounted separately to the support, such that one or more cameras could be worn about the head or body of the wearer via a clip or Velcro attachment etc. Indeed, additional cameras could also be used in conjunction with spectacle frame mounted cameras, for example, the wearer could have a rear-facing camera which supplements the information from the front-facing cameras, so that any approaching object from the rear could be brought to the attention of the wearer.

The optical device preferably further comprises a computing device for controlling the array(s) and/or the image capture device. The computing device is preferably a portable computer comprising at least a processor and a memory. By "portable" we mean that the computer is preferably a self-contained unit that may be worn about the body of the wearer and carried with the wearer as he/she navigates and negotiates their environment.

In preferred embodiments, the computer is separately wearable to the spectacle frame, and in one embodiment may be clipped to a belt of the wearer or alternatively be worn in a sling-like harness across the body of the individual. Of course, any suitable mechanism for attaching the computer to the wearer may be used in conjunction with the invention.

The computer is preferably coupled to the arrays and the cameras by way of wired electrical connections. However, in other embodiments, a wireless connectivity could be adopted between the components of the device. However, in the interests of preserving power and/or prolonging operational use, it is envisaged that wired connections will be used for most applications.

Preferably, the computer is powered by an internal battery, which may be rechargeable. In preferred embodiments, the LED arrays and the cameras will also be powered by the computer's battery. However, the spectacle frame itself could be provided with its own power source, such as cell or battery, but of course this would increase the overall weight of the device which is not especially desirable. In other embodiments, a separately wearable 'battery pack' could be worn by the individual to provide power to the spectacle components.

In preferred embodiments, an image processing means is implemented in the computing device. The image processing means may be a software module that is executed on the processor or alternatively this may be configured as a hardware component in the portable computer. In cases where the image processing means is a hardware component, it may comprise its own processor or else make use of the main processor of the portable computer. Of course, any suitable arrangement may be adopted, and indeed a mix of software and hardware components may also be used depending on the particular application.

The image processing means is preferably operable to identify and locate objects in the images captured by the image capture device. By "objects" we mean any distinguishable entities or shapes within the images that correspond to, but are not limited to, physical or natural structures (e.g. walls, floors, doorways, trees etc.), obstacles (e.g. tables, chairs, lampposts, cars), items (e.g. telephones, mugs, foodstuffs etc.), people (e.g. human faces), words, phrases and text (e.g. signage, shop & retail names, newspaper headlines, informational boards etc.).

In preferred embodiments, the identification of objects is achieved by applying one or more algorithms to the captured images to preferably search for predetermined shapes or forms in the images which are likely to correspond to known object or object types. Hence, the identification algorithm is preferably configured to determine if any known objects are present in the captured images, and if so, to preferably identify one or more of the object type, spatial size, its position relative to the individual and distance to the object.

The presence of objects are preferably determined by reference to a database or library of stored shapes and forms, which preferably forms part of the computing device, and may be stored in memory. The database of stored shapes is preferably classified by differing object properties and characteristics, such as shape, distinctive contours and colour etc. Therefore, if an identification algorithm detects a shape in a captured image, for example by delineating a contour or continuous edge associated with that shape, the shape is then compared to the stored object recognition files and a match is attempted to be found.

Hence, for example, if the wearer is next to a table having a teapot on top of the table, the image processing means is able to locate the object in a captured image of that scene and identify the object as a teapot by reference to the database of stored shapes. It is envisaged that the database will comprise a large number of objects commonly encountered in every day life. However, inevitably some objects will not be known to the image processing means, or else cannot be adequately identified (e.g. due to other foreground/background object interference or obscuration etc.), and so in such circumstances a match may not be possible. In such an event, the wearer may then be informed that an unidentified object is nearby, and may possibly be instructed to re-image the object from a different angle (e.g. by changing their relative position). In preferred embodiments, the device is also able to learn new objects by way of an inherent learning function (as will be discussed later).

In much the same way, human faces may also be identified by the image processing means. Preferably, a facial recognition algorithm is also applied to the captured images and if another person is within the immediate vicinity of the wearer (and their face is not obscured) the algorithm can notify the wearer that a person is nearby. In preferred embodiments, facial recognition is achieved using a two-stage process. The first stage preferably performs colour matching from the captured images with a set of pre-stored skin coloured swatches. In this way, a attempt is made to identify any colours that match a recorded skin tone (e.g. caucasian or other ethnicity etc.). While the second stage preferably limits any detection results with a sufficient degree of sphericity, corresponding to a typical facial shape. To further improve the reliability of the facial recognition, a facial feature algorithm may also be applied to the images, which searches the spherical object for indications of eyes, a nose or a mouth etc.

In addition to identifying objects and recognising faces etc., the image processing means is also preferably able to estimate distances to the identified objects and to convey this to the wearer of the device. In preferred embodiments, the distance of an object may be calculated via parallax, which is determined by analysing the apparent angular shift of the object relative to background features in each of the images captured by the pair of wide-angle cameras. Therefore, since the separation between the two cameras is known (and is fixed), determining the angle of parallax then gives a reliable estimate of the distance of the object by way of a simple trigonometric calculation, which can be performed by the processor. An alternative approach, which may be used in other embodiments or in combination with parallax shift techniques, is to build up a simple map of the identified surfaces using a distance estimation algorithm such as PTAM (Parallel Tracking and Mapping) developed by G. Klein and D. Murray at Oxford University. The algorithm identifies surfaces and edges in the images and can estimate the distances to the surfaces via stereoscopic techniques based on the different viewing angles of the wide-angle cameras. By translating the spectacle frame, by movement of the wearer and the wearer's head, the algorithm can be initialised and a map of the estimated depth distribution can be generated. In this way, it is then possible to represent this map as a distance-brightness scale on the LED arrays, with nearer surfaces being represented by brightly illuminated LEDs and more distant surfaces being represented by relatively dimmer illuminated LEDs. As distance determination is an important aspect of many of the embodiments, it is envisaged that a specific colour, for example while light, will be used to convey distance information.

Of course, it is to be appreciated that any suitable technique of distance determination may be used with the optical device of the present invention. Therefore, in other embodiments, infra-red (IR) or ultrasonic ranging devices may alternatively, or additionally be utilised. Such devices could be integrated into the support itself, or else may be separately wearable by the individual.

In preferred embodiments, the computer is able to collate all of the information (e.g. objects, distances etc.) gathered from the captured images and to determine how this information is to be conveyed to the wearer of the device. As mentioned earlier, in all embodiments particular patterns of illumination may be assigned to specific objects or object types that have been identified in the images. In some embodiments entire classes of objects may be represented as a single pattern and/or by a single colour or texture. Therefore, faces, text and distances may form individual classes which are indicated to the wearer by way of a different pattern of illumination and/or colour.

Hence, taking the example of an indentified face in the wearer's immediate environment, the computer may send signals to the LED arrays that cause at least one of the arrays to illuminate a circle of LEDs, or otherwise a swatch of colour, to represent a human face. Moreover, depending on the size of the circle or swatch of colour, this could give an indication as to the approximate distance of the person. Hence, a small illuminated circle of LEDs could imply the person is some distance away from the wearer, while a larger circle could imply that the person is relatively closer to the wearer. Thus, it follows that an increasing circle could indicate that the person is approaching the wearer, while a decreasing circle could indicate that the person is receding from the wearer.

In addition, an approximate indication of the position of the person relative to the wearer may also be provided by illuminating the circle in either the left or right hand display lens/array, so that the wearer knows that the person is towards their left or their right depending on the position of the illuminated circle.

For individuals where their visual-impairment would not allow an illuminated circle to be discerned, any other suitable pattern of illumination could alternatively be used. Therefore, a cluster of adjacent LEDs could instead be illuminated, so that only a single swatch of light is detected by the wearer. The LED cluster may also be modulated so that the light flashes at a predetermined rate (e.g. 1 Hz), and/or colour, to indicate that a face has been identified. Thereafter, the frequency of modulation could be increased if the person moves towards the wearer, or else decreased if the person moves away from wearer etc.

It can be appreciated therefore that any appropriate pattern of illumination and/or colour, whether that be spatial (e.g. distributed across the array or localised as sub-sets of LEDs) or temporal (e.g. single or multiple LED 'flashing' modulation) may be used to convey information relating to objects and/or distances in the wearer's environment to the wearer of the optical device. Indeed, in some examples it has been possible to manipulate both the rate of flashing as well as combinations of vertical and horizontal flicker in the arrays, so as to generate substantially 'checkerboard' patterns for use to discriminate between object classification. Hence, via appropriate assignment of illumination patterns to general or specific object types, together with suitable training for the wearer, the optical device of the present invention can provide significant assistance to a visually-impaired individual in navigating and negotiating their immediate environment.

In addition, in some preferred embodiments, the image processing means is further operable to perform text recognition based on any textual content in the images captured by the image capture device. Therefore, the image processing means preferably comprises an algorithm for carrying out optical character recognition (OCR) on any identified words, phrases or signage in the images of the wearer's immediate environment. Preferably, customised character sets are stored in the computing device, which act as a library for the OCR algorithm. In preferred embodiments, the text recognition is carried out as a multi-stage process that initially involves detecting letters in the library of character sets. The orientation of the characters is preferably estimated, and the successive characters are built up along the orientation lines. Each successive captured image is preferably analysed for known letters, with error and fidelity checks preferably being performed by a simple mode filter. Any gaps are estimated and are used to segregate potential words, which are then preferably compared to a stored lexicon. The completed words may then also be mode filtered, preferably via several repetitions, to generate the most likely phrase or sentence etc.

In some embodiments, the character sets may comprise data concerned with public transport (local bus numbers and routes, underground stations etc.), supermarket price tags and newspaper headlines etc. Any of the character sets may be customised to the wearer's local environment to further aid ease of mobility and navigation.

Specific words or phrases, such as those relating to warnings (e.g. stop signs, hazard signs etc.) may be assigned a unique pattern of illumination in the array. Hence, should the OCR algorithm detect the word "DANGER" in an image of the immediate environment of the wearer, both arrays may be made to repeatedly flash, preferably red, until the wearer has navigated away from the potential hazard.

Preferably, the computing device also comprises a speech synthesiser that is operable to provide a spoken output corresponding to the text recognised by the OCR algorithm.

The spoken output is preferably provided in real-time to the wearer of the device, so that instructions, warnings or other information can be notified to the wearer to aid their navigation and provide feedback on their immediate environment. Hence, the optical device preferably comprises an audio output device, such as a pair of headphones that may be integrated into, or otherwise attached to the support, for example the arms of the spectacle frames. Alternatively, the headphones may be separate components that connect to an audio output jack on the computing device.

The optical device also preferably comprises a control interface to control the operation of the device. The control interface is most preferably voice-activated, such that the wearer is able to issue spoken or verbal commands to the device in order to initiate or inhibit some particular function. Preferably, the control interface comprises a microphone that is operable to receive the spoken commands. The microphone may be a miniature type microphone that is preferably mounted to the support, which in the case of a spectacle frame is preferably on the inside of the frame behind one of the display lenses/arrays. Of course, the microphone may be situated at any other suitable location, and may alternatively be a separate component to that of the support, and thus can be clipped or attached to the wearer's apparel etc.

Any operation of the optical device may be controlled via the control interface including, but not limited to switching the device ON or OFF; instructing the object identification algorithm to ignore certain objects or object types; to switch the speech synthesiser ON or OFF (to commence or inhibit the output of spoken words recognised in the images); and to commence or terminate recording of a sequence of images (for later processing - as discussed below in relation to the inherent learning function).

A clear advantage of using a voice-activated control interface is that the visually-impaired wearer does not need to manipulate any switches or controls on the support or computing device, which thereby further improves the ease of operation and use of the device.

In preferred embodiments, the computing device further comprises an adaptive learning means that is operable to learn different objects so as to discriminate between different object types. In addition, the adaptive learning means may also learn to recognise new text (e.g. words, phrases etc.) based on the textual content in the captured images.

The adaptive learning means is preferably implemented in software and in preferred embodiments has two modes of learning that allow it to save new objects into the database or library of objects, which is used by the identification algorithms to identify objects in the images. The first mode is preferably wearer-initiated, such that objects can be presented to the optical device and the wearer can instruct the device to 'learn' the new object. Hence, for example, the wearer may hold up a can of soft-drink and then issue the spoken command "LEARN", which preferably triggers the adaptive learning means to record a video sequence via the image capture device. The recorded video sequence my then be analysed to build up an object recognition file for that new object, and in some embodiments may have additional functionality to allow a category to also be assigned to that object, for example "DRINK".

The analysis of the recorded video sequence may be performed either 'OFFLINE' (e.g. while the optical device is not in active use by the wearer) and preferably remotely from the optical device. It is envisaged that the recorded video sequences may be uploaded to a remote secure server, as maintained by the equipment manufacturer or developer etc., or else to the wearer's personal computer (e.g. desktop or laptop etc.). The need for a 'secure' server is to allay any concerns of the wearer regarding the uploading of their personal video sequences. Therefore, the video files may also be encrypted to prevent unauthorised viewing of the sequences, and would preferably be automatically deleted from the server after analysis had been completed.

An advantage of carrying out the analysis remotely to the device is that this reduces processing overheads on the processer of the computing device, which could diminish performance of the optical device during use, or else shorten battery life etc. In either case, software will preferably perform the object recognition and generate a object recognition file for subsequent download to the database or library of the optical device. In this way, new objects can be continuously or periodically added to the database or library, building up a customised collection of object recognition files for the wearer.

In other embodiments, the processing of the video sequence could however be carried out gradually during use of the device, by making use of any spare processing cycles of the internal processor or by exploiting any 'idle time' when the device and/or software is not currently carrying out an operation etc. Alternatively, the processing could be performed when the device is not in use and is recharging.

The second learning mode is preferably a behavioural led form of learning, such that the behaviour of the wearer can be monitored and deduced in order to preferably update the object recognition database or library. In preferred embodiments, the support further comprises an orientation determining means to determine the orientation of the support relative to the individual's immediate environment. Preferably, the orientation determining means is in the form of a gyroscope, and most preferably a tri-axial gyroscope, which is primarily intended to aid stabilisation of the video images. However, the output of the gyroscope may also be used to perform an approximate estimate of the wearer's ongoing behaviour. For example, if the device is functioning and the gyroscope indicates that the wearer is stationary, then it is reasonable to assume that the wearer is engaged in a meaningful task. If the object recognition algorithms do not recognise any objects or text in the captured images, the adaptive learning means can then preferably be set to automatically begin recording a video sequence for subsequent object recognition (either offline and/or remotely etc.). Thus, any objects associated with that meaningful task that are not yet in the database or library can be analysed and appropriate object recognition files can be generated and saved for use in future objection identification.

In alternative embodiments, the orientation determining means may be an accelerometer.

According to a second aspect of the present disclosure there is provided an optical device for a visually-impaired individual, comprising
a compound display comprising first and second arrays of a plurality of addressable light-sources;
a support arranged to maintain the arrays in proximate relation to at least one eye of the individual, such that the second array is angled relative to the first array; and
an image capture device configured to capture images of at least part of the individual's immediate environment;
wherein the first and second arrays are configured to provide an optical stimulus to the individual's central and/or peripheral vision by selectively illuminating one or more of the addressable light sources based on the content of the captured images to thereby convey information to the individual.

In this aspect, the optical device is configured to comprise a compound display that is arranged to provide an optical stimulus to the wearer's central and/or peripheral vision by way of first and second arrays of a plurality of addressable light-sources. By 'central vision' we mean the wearer's vision substantially along his/hers line of sight (typically looking forward or ahead), while 'peripheral vision' is intended to encompass any lateral or side of the eye visual function, and typically relates to the wearer's vision at an angle to their direct line of sight.

The first array is preferably different to that of the second array, and in particular, the first array preferably comprises a greater number of addressable light-sources than the second array. It has been found that during testing, some visually impaired wearers retained sufficient visual resolution to be able to discern the spacing between the light sources in the embodiments of the first aspect of the disclosure. Therefore, for such individuals, a higher resolution display may be more beneficial. Hence, in the compound display of the second aspect of the invention, the first array preferably corresponds to a higher resolution array as compared to the second array, which may be similar in form to the spaced LED array of the earlier embodiments.

In particularly preferred embodiments, the first array may be an OLED (organic light-emitting diode) 2D display comprising individually addressable LEDs. OLED display technology is commonly used in mobile phones, due to its compact-size, low weight, low cost and low power requirements. In particular, considerable research and development has been directed towards developing transparent OLED displays, which are particularly suitable for use with the present device.

Therefore, even with the use of OLED display technology, it is still possible to fabricate lens type inserts for a spectacle support, as described in relation to the earlier embodiments, without sacrificing any of the advantages of the present invention.

The second array may be the same as the spaced LED array as described above for the embodiments of the first aspect.

However, in most cases it is envisaged that this will be reduced in scale (i.e. a smaller version of the array) so that it is better suited for use with this aspect of the invention. Therefore, in preferred arrangements, a spaced LED array will be disposed adjacent to a respective one of the arms of the spectacle frame support, with the array being angled to the OLED array to permit the wearer's peripheral vision to be optically stimulated by selectively driving one or more of the spaced LEDs.

Hence, in this configuration, the wearer's central vision may be stimulated by the higher resolution (transparent) OLED display, while their peripheral vision may be stimulated by the lower resolution spaced LED array. This arrangement has significant advantages, not least, in terms of the increased informational content that can be conveyed to the wearer, by way of the combined use of two separate displays for each respective eye.

As described above in relation to the embodiments of the first aspect a fundamental difference between the present disclosure and known visual aids, is that the information presented to the wearer by the present device represents the distance to objects within the wearer's environment and not the features of the objects themselves. Consequently, it is not necessary for the visually impaired wearer to possess or retain any focussing ability, as the objects themselves do not need to be discerned. In other words, rather than zooming in or enhancing a scene in front of the wearer, the present device preferably makes use of a pair of cameras to stereoscopically generate a 2D 'depth image' or 'depth map', such that nearby objects can be represented by bright regions of light, while more distant objects can be shown as darker regions of light, gradually fading way to black.

In addition to the use of transparent OLED type displays, further modifications and/or enhancements may be made to any of the embodiments described in relation to either the first or second aspects of the present disclosure.

Therefore, as alluded to earlier, the present device may also include an ultrasonic range finder, which is preferably mounted above, on or proximal to the bridge of the support frame. The function of the range finder would be to preferably detect objects less than about 1 metre away from the wearer and to provide a substantially 'fail-safe' mechanism to avoid collisions with objects that are undetectable by the pair of cameras, for example, glass doors etc. Information gathered from the ultrasonic range finder would be conveyed to the wearer using the displays as described above, namely by providing a spatial and/or temporal pattern of selective illumination, preferably consistent with the use of the depth image or map. Hence, in exemplary embodiments, the central portion of the display would become brighter as objects approached the wearer or as the wearer approached the objects.

As discussed above, in addition to the support frame comprising a gyroscope, the frame may also include any or all of an accelerometer, electronic compass and a GPS receiver. Data from the gyroscope and accelerometer may be combined using statistical algorithms, such as a Kalman filter, which enables the orientation of the frame to be calculated. Having knowledge of the frame's orientation can be useful, not least in that, it can be used for the following purposes:
1. Assisting the image processing - frames collected during rapid head movement may be excluded from the image processing due to excessive blurring, which may reduce processing time and potentially save battery power. Moreover, background subtraction of the image can be performed if the movement of the camera is known, which is very useful for detecting people within the images.
2. The visual display can be modified based on the orientation of the camera. For example, it is possible to remove the 'floor surface' from the image displayed to the wearer to assist the wearer with identifying objects on the ground, together with steps or stairways etc. Knowing the orientation of the cameras helps the processing software to identify the plane of the ground.
3. Augment the visual display - the update speed of the display may be improved by interpolating the position of objects in the display based on the movement of the cameras.

The GPS and compass may be used to locate the wearer on a digital map and assist in so called "wayfinding". Wayfinding involves providing visual directions to navigate towards a remote target location. Once the wearer is located via the GPS, the computer will calculate a route to their destination, and will convey instructions to the wearer, via the displays, to direct them along the route. Hence, the present device may provide a virtual 'line' to follow, with re-orientation signals, such as bright indicators on the left or right hand side of the displays, should the wearer stray or deviate from the virtual line.

In another application, the GPS and compass may also be used to provide public transport assistance. For example, if the wearer notifies the device that he/she intends to catch a bus, then the software can attempt to determine the wearer's position, while identifying the nearest bus stops to the wearer. In addition, the software can obtain information on bus routes and timetables, and can audibly inform the wearer of the time of the next bus and route numbers etc. by way of the device's headphones. The real-time bus arrival information may be used to aid the object and character recognition algorithms, which will attempt to detect the route number of oncoming buses. A similar arrangement may be used for rail services and train times etc. where such information is posted in real-time to the Internet. As such, the present device may incorporate hardware and/or software for connecting to the Internet via wi-fi or mobile phone networks (e.g. 3G) etc.

To further enhance the delivery of the public transport information, the device may also be configured to provide the wearer with a spatially relevant (e.g. directional) audio, which can convey to the wearer a sense of directionality, in that the wearer understands the direction from which the bus or train is approaching etc. The audio is preferably a 2D audio, but any suitable mixed channel audio may be used to convey a sense of direction. Hence, for example, during use the device may detect an approaching bus, which via application of an OCR algorithm, enables the number of the bus (or route etc.) to be determined, The device can then audibly convey this information to the wearer, via a speech synthesiser, with the audio being adapted to account for the wearer's head position and/or direction, such that the speech appears to be coming from the direction of the approaching bus. In this way, the directionality of the speech can provide a more consistent and realistic sense of space for the wearer, while also potentially improving safety, as the wearer knows the direction from which the bus is approaching.

To avoid the wearer feeling audibly or acoustically isolated from their environment, particularly during wayfinding or travelling on public transport, miniature microphones or transducers may be incorporated into the head phones (e.g. ear buds of the head phones) of the device to allow at least some ambient sounds to be conveyed to the wearer. This arrangement may be used in conjunction with any of the embodiments of the present device and would be selectively controllable by the wearer, so that the transmitted ambient sounds could be turned on or off as desired.

In addition to the manual and/or audible (e.g. voice recognition) control of the present device, as discussed above, a further enhancement may be based on detecting facial gestures of the wearer. Therefore, in some embodiments a set of electrodes may be attached around the orbit of the eye (e.g. the circumference of the eye socket) in order to measure electrical potentials on/in the skin. Such electrodes can detect simple eye movements, for instance, winking and raising/lowering eyebrows etc., with these actions being used to control properties of the display, such as zooming in or out etc.

A further option to control the device and/or properties of the display may be also achieved by way of 'head gestures', such that movements of the wearer's head (e.g. raising or lowering their head, moving their head side to side relatively quickly etc.) could be used to switch visual and/or audio functions on or off etc. Therefore, the accelerometer may provide information to the software, which allows the software to change a property of the display, for example, by zooming in or out. The head gestures may be used in combination with the facial gestures to perform a whole range of tasks and to control the operation of the device. Of course, it is to be appreciated that any suitable head movement and/or facial gesture may be used to control and operate the device.

In preferred embodiments, the device may also include a light sensor, such as a light dependent resistor (LDR), to monitor ambient light levels in the wearer's local environment. In this way, the sensor may be used to automatically control and adjust the brightness of the display to suit the lighting conditions.

To ensure that the pair of cameras are able to detect objects in low level light, the device may also comprise a set of infra-red (IR) LEDs, which may be turned on when the light sensor indicates that the level of lighting has fallen below a predetermined threshold.

In order to supplement and complement the function of stereoscopic depth imaging provided by the pair of cameras mounted on the frame, a structured light emitter may also be integrated into the support frame of the device. The structured light emitter may be a low-powered infra-red laser, most preferably a laser diode, that projects a holographic diffraction pattern via a two-dimensional diffraction grating at the exit aperture of the diode. The laser and grating combination produces a large field of tightly spaced dots, which may be used to provide sufficient features in the image to perform depth calculations. It is found that this feature works particularly well for large flat and featureless objects, such as plain white walls etc.

The laser diode is preferably mounted above the bridge of the support frame and may be powered by way of the device's battery.

For eye conditions such as age-related macular degeneration it is generally useful to be able to track the eye position of the wearer in order to be able to direct the image to the optimal part of the visual field. Hence, for example, if the wearer has residual vision on the far left and far right of the visual field, then the software is arranged to re-orientate the display to ensure that the information is provided in these two regions. However, if the wearer moves their eyes, the display regions may then fall outside of their residual vision, which is why it is necessary to continually track the wearer's eye position to dynamically adjust the display accordingly. In preferred embodiments, eye tracking may be achieved by using a single miniature camera, fitted with a macroscopic lens and tuned to detect only infra-red (IR) light. The camera would be preferably paired with an infra-red (IR) LED, which would shine onto the wearer's eye thereby enabling the movement of the eye to be tracked. An iris detection algorithm is preferably applied to the video stream from the camera, which allows the current direction of the wearer's gaze to be determined.

Although the present device is ideally suited to assist visually impaired individuals to negotiate and navigate their environment, the device may also be used for enhancing entertainment experiences, such as watching television. As discussed, the device is not designed to improve the image of the wearer's scene per se, but to provide information relating to the location of objects within the scene. Therefore, it is possible to use the device to indicate the approximate location of people and objects within a television picture or image, and potentially even sportspeople in a sporting event, such as a football match etc. In preferred embodiments, a person detection algorithm and a face detection algorithm may be applied to a pre-recorded video of a television programme. The algorithm thereby records the location and possibly identity (with prior training) of the faces in the programme and can subsequently provide that information as a 'close-caption subtitling' type data stream etc. Consequently, the wearer, while listening to the audio in the television programme, can receive the character data stream which thereby indicates to them the position of key faces in the television scene via colour coded patterns or flashing regions of light etc. Hence, in this way the wearer can obtain a better appreciation of the television scene, which consequently enhances their enjoyment of the programme as they are able to 'see' the spatial interaction between the characters and any subsequent movement in the scene.

It is envisaged that a similar technique could be applied to video of football matches, with the wearer being presented with a simulated (top-down) view of the pitch generated by an appropriate image algorithm. Hence, while listening to the match commentary, the position of the ball and key players (e.g. those currently 'in-play') could be indicated on the simulated pitch, with any unknown player positions being shown as a standard formation (e.g. 4-3-3 or 4-4-2 etc.) appropriate to that team and game.

It is to be understood that none of the preceding embodiments are intended to be mutually exclusive, and therefore features described in relation to any particular embodiment may be used additionally and/or interchangeably with features described in relation to any other embodiment without limitation.

The invention is defined in the claims. Other embodiments are merely exemplary. 25

Embodiments of the present invention will now be described in detail by way of example and with reference to the accompanying drawing in which:
**Figure 1** **-** is a schematic representation of an optical device according to a preferred embodiment of the present invention.
**Figure 2** **-** shows a front/side perspective view of a part of an optical device according to a particularly preferred embodiment of the present invention;
**Figure 3** **-** shows an above/reverse perspective view of the part of the optical device of Figure 2;
**Figure 4** **-** shows a side/reverse perspective view of the part of the optical device of Figure 2;
**Figures 5A** & 5B - show respective reverse/front perspective views of an optical device according to another preferred embodiment of the present invention.

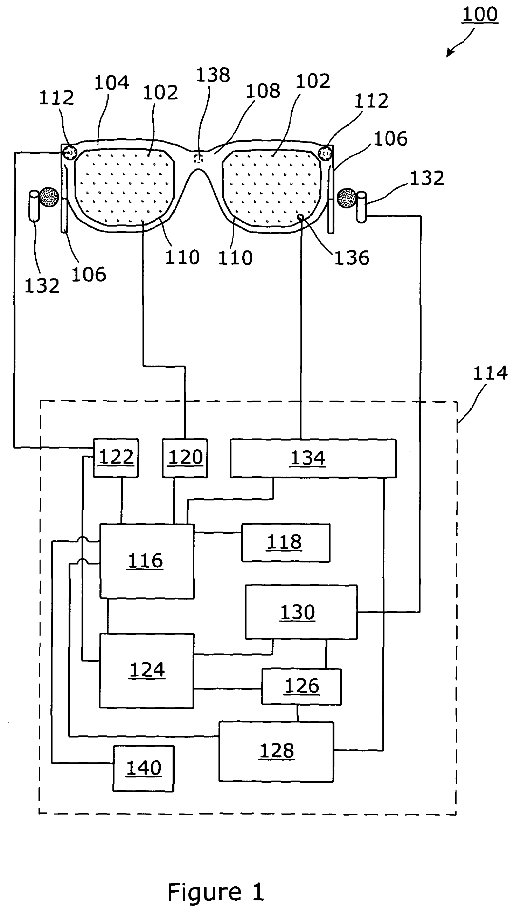

Referring to Figure 1, there is shown a particularly preferred embodiment of an optical device 100 according to the present invention. The optical device 100 comprises a spaced array of discrete light sources 102 and a support 104 arranged to maintain the array 102 in proximate relation to at least one eye of a visually-impaired individual (not shown).

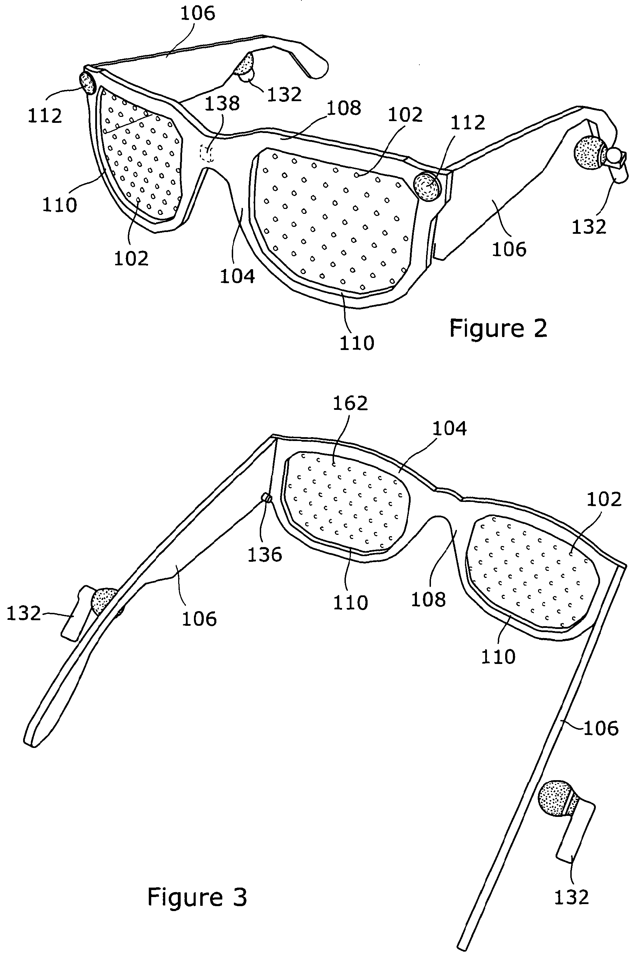

In the example of Figure 1, the support 104 is in the form of a spectacle frame made from a rigid plastic material. The spectacle frame 104 comprises two foldable arms 106 (better shown in Figures 2 to 4) and a bridge portion 108 having two respective lens sockets 110. The spaced array 102 is implemented as two separate 'displays', each in the shape of a spectacle lens which is fitted into a respective lens socket 110 in the frame 104. In this way, one display is presented to each respective eye of the wearer of the optical device 100.

As shown in Figures 1 to 4, the discrete light sources are composed of a matrix of individually addressable light emitting diodes (LEDs), which are distributed across the surface of the lens to form a display of approximately 35 x 30 mm in size. In the examples of Figures 1 to 4, there are around 50 separate LEDs (measuring approx. 2 x 1 mm each) in each array 102, which are spaced from each so as to form an approximate 8 x 6 landscape hexagonal configuration.

The LEDs may be a pure white colour or else be coloured (e.g. red and/or green) or a combination of both, and any of single, dual and/or multi-coloured diodes may be used.

The lenses themselves act as mere supports for the arrays 102 of LEDs and consequently provide no optical correction to the wearer's vision. The lenses are made from a plastic material, which in the examples of Figures 1 to 4 is transparent, but opaque lenses may alternatively be used. The use of transparent lenses can be useful to certain visually-impaired individuals, as they may still rely on 'background light' detection to help with mobility and navigation. Therefore, in some situations it may not be desirable to block or diminish any background light when using the present optical device.

Although not shown in any of the figures, the LEDs have been integrated into the moulded plastic material of the lenses, together with their respective electrical connections (which are not shown for clarity purposes). However, the LEDs may be applied directly to the inner or outer surfaces of the lenses, via adhesive etc., or can be mounted on a transparent conductive film, which can then be overlaid onto a surface of the lens.

Referring again to Figure 1, the optical device 100 further comprises an image capture device in the form of two wide-angle video cameras 112. The video cameras 112 are respectively mounted at the upper corners of the frame 104, each above a respective lens socket 110. In this way, the captured images track or follow the wearer's line of sight, so that when the wearer turns his/her head the cameras 112 image whatever is located along that particular direction. The video cameras 112 are miniature colour video cameras of the CMOS variety, with wide-angle lenses of apparent field of view of 120 degrees, although any small, lightweight camera may alternatively be used.

An advantage of using two spaced apart cameras is that distance information can be determined via stereoscopic techniques by virtue of the different viewing angles of the cameras. Therefore, the function of the cameras 112 is to capture video sequences of the wearer's immediate environment so that object location and identification can be carried out in order to provide the wearer with information about his/her surroundings. In this way, information relating to objects, obstacles and distances can be conveyed to the wearer by selectively illuminating one or more of the LEDs in the arrays 102 according to predetermined patterns of illumination and/or colour.

The frame 104 is dimensioned such that the arrays 102 are held at a distance of between around 3 to 5 cm from the wearer's eye. In most cases, this will normally be less than the minimum focal length of the eye (i.e. the shortest distance at which focus can theoretically be attained). However, that does not matter in the present invention, and indeed this feature provides a significant advantage, as it is not necessary for the wearer to focus on the LEDs in the array - unlike in conventional augmented-reality devices that require the individual to resolve parts of an enhanced image. Therefore, the present optical device is able to convey information to visually-impaired wearer's by making use of their residual visual function, irrespective of whether they are able to focus on images or not.

However, another advantage of placing the arrays 102 close to the eye is that the intensity of the light received by the wearer's eye can be increased, which potentially enhances the perception between light and dark.

Referring again to Figure 1, the optical device 100 further comprises a computer 114 (shown as ghost lining) which is arranged to control the functions and operation of the device, and in particular the arrays 102 and cameras 112. Although not explicitly shown in Figure 1, the computer 114 is intended to be separately wearable to the spectacle frame 104, and may be clipped to a belt of the wearer or alternatively be worn in a sling-like harness etc. across the wearer's body. Of course, any suitable mechanism for attaching the computer 114 to the wearer may be used in conjunction with the present invention.

The computer 114 comprises at least a processor 116 and a memory 118, and is coupled to the arrays 102 via a driver 120 and to the cameras 112 via video buffer 122. (In the interest of clarity only single connections are shown in Figure 1 to one array 102 and one camera 112, however it is be understood that in practice both arrays and both cameras are coupled to the computer 114). The driver 120 may, for example, be a PIC controller that provides buffering for each individually addressable LED in the arrays 102. The video buffer 122 may be any suitable video buffer device.

An image processing means 124 is also implemented in the computer 114, which is operable to identify objects in the video images captured by cameras 112. The image processing means 124 may be a software module that is executed on the processor 116, or be a hardware component which utilises processor 116 and/or memory 118. Alternatively, the image processing means 124 may be implemented in both software and hardware. In any event, the function of the image processing means 124 is to identify and locate objects in the images captured by the cameras 112.

By "objects" we mean any distinguishable entities or shapes within the images that correspond to, but are not limited to, physical or natural structures (e.g. walls, floors, doorways, trees etc.), obstacles (e.g. tables, chairs, lampposts, cars), items (e.g. telephones, mugs, foodstuffs etc.), people (e.g. human faces), words, phrases and text (e.g. signage, shop & retail names, newspaper headlines, informational boards etc.).

The identification of objects is achieved by applying one or more algorithms to the captured video images to search for predetermined shapes or forms in the images which are likely to correspond to known object or object types. Hence, an identification algorithm is configured to determine if any known objects are present in the captured images, and if so, to identify one or more of the object type, spatial size, its position relative to the wearer and distance to the object.

The presence of objects are determined by reference to a database 126 of stored shapes and forms, which is implemented within the computer 114. The database 126 is classified by differing object properties and characteristics, such as shape, distinctive contours and colour etc. Therefore, if an identification algorithm detects a shape in a captured image, for example by delineating a contour or continuous edge associated with that shape, the shape is then compared to the stored object recognition files and a match is attempted to be found.

The database 126 comprises object recognition files for a large number of objects commonly encountered in every day life. However, inevitably some objects will not be known to the image processing means 124, or else cannot be adequately identified (e.g. due to other foreground/background object interference or obscuration etc.), and so in such circumstances a match may not be possible. In such an event, the wearer is then informed that an unidentified object is nearby, and may possibly be instructed to re-image the object from a different angle (e.g. by changing their relative position). However, the optical device 100 is also able to learn new objects by way of an adaptive learning module 128 so that the database of object recognition files can be updated over time (as discussed below).

In much the same way, human faces are also identified by the image processing means 124. Therefore, a facial recognition algorithm is also applied to the captured images and if another person is within the immediate vicinity of the wearer (and their face is not obscured) the algorithm notifies the wearer that a person is nearby. The facial recognition is achieved using a two-stage process. The first stage performs colour matching from the captured images with a set of pre-stored skin coloured swatches. In this way, a attempt is made to identify any colours that match a recorded skin tone (e.g. caucasian or other ethnicity etc.). While the second stage limits any detection results with a sufficient degree of sphericity, corresponding to a typical facial shape. In other examples, a facial feature algorithm is also applied to the images, which searches the spherical object for indications of eyes, a nose or a mouth etc.

In addition to identifying objects and recognising faces etc., the image processing means 124 is also able to estimate distances to the identified objects and to convey this to the wearer of the device 100. The distance of an object is calculated via parallax, which is determined by analysing the apparent angular shift of the object relative to background features in each of the images captured by the pair of wide-angle cameras 112. Therefore, since the separation between the two cameras 112 is known (and is fixed), determining the angle of parallax then gives a reliable estimate of the distance of the object by way of a simple trigonometric calculation, which is carried out on the processor 116.

In an alternative approach, a simple map of the identified surfaces is instead built up using the distance estimation algorithm called PTAM (Parallel Tracking and Mapping), developed by G. Klein and D. Murray at Oxford University (http://www.robots.ox.ac.uk/~gk/PTAM/). The algorithm identifies surfaces and edges in the images and estimates the distances to the surfaces via stereoscopic techniques based on the different viewing angles of the wide-angle cameras 112. The algorithm is initialised by translating the spectacle frame 104, which can be achieved by the wearer moving their head and position. In this way, a map of the estimated depth distribution is then generated, which is represented as a distance-brightness scale on the LED arrays 102. As distance determination is an important aspect of the information conveyed to the wearer, this is represented by white light in the arrays 102, with closer surfaces to the wearer being brighter than surfaces which are further away.

In addition, the image processing means 124 is further operable to perform text recognition based on any textual content in the images captured by the cameras 112. Therefore, in the example of Figure 1 the image processing means 124 further comprises an algorithm for carrying out optical character recognition (OCR) on any identified words, phrases or signage in the images of the wearer's immediate environment.

Customised character sets are stored in the database 126, which act as a library for the OCR algorithm. Text recognition is carried out as a multi-stage process that initially involves detecting letters in the library of character sets. The orientation of the characters is estimated, and the successive characters are built up along the orientation lines. Each successive captured image is analysed for known letters, with error and fidelity checks being performed by a simple mode filter. Any gaps are estimated and are used to segregate potential words, which are then compared to a stored lexicon. The completed words may then also be mode filtered, via several repetitions, to generate the most likely phrase or sentence etc.

The computer 114 is able to collate all of the information (e.g. objects, distances etc.) gathered from the captured images and to determine how this information is to be conveyed to the wearer of the device 100. As mentioned earlier, particular patterns of illumination and/or colour are assigned to specific objects or object types that have been identified in the images. Therefore, entire classes of objects are represented as a single pattern and/or by a single swatch of colour or texture. Therefore, faces, text and distances have been chosen to form individual classes which are indicated to the wearer by way of a different pattern of illumination and/or colour.

It can be appreciated therefore that any appropriate pattern of illumination and/or colour, whether that be spatial (e.g. distributed across the arrays 102 or localised as sub-sets of LEDs) or temporal (e.g. single or multiple LED 'flashing' modulation) may be used to convey information relating to objects and/or distances in the wearer's environment to the wearer of the optical device.

As shown in Figure 1, the computer 114 also comprises a speech synthesiser 130 that is operable to provide a spoken output corresponding to the text recognised by the OCR algorithm. The spoken output is provided in real-time to the wearer of the optical device 100, so that instructions, warnings or other information are notified to the wearer to aid their navigation through their immediate environment. Hence, the optical device 100 comprises an audio output device in the form of a pair of headphones 132 that is integrated into, or otherwise attached to the arms 106 of frame 104, as shown in Figure 2. (In the interest of clarity only a single connection is shown in Figure 1 to one speaker of headphones 132. However it is be understood that in practice both speakers are coupled to the speech synthesiser 130).

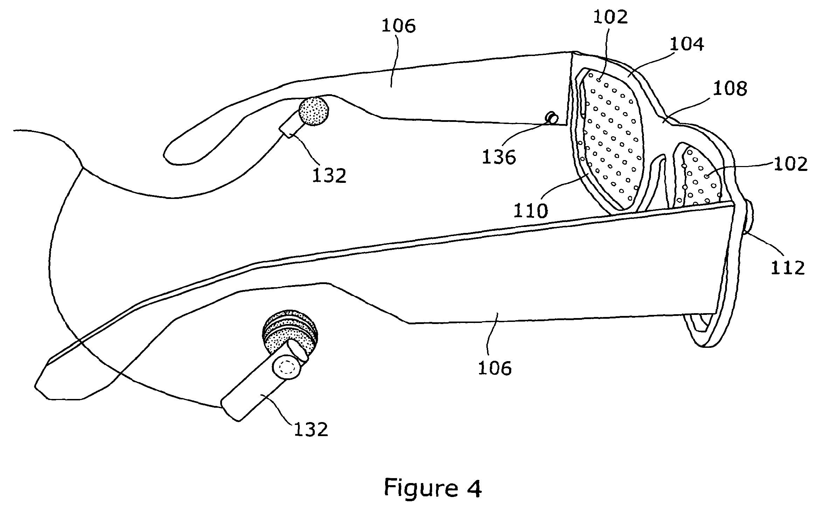

In other examples, the headphones 132 can be separate components to the frame 104, as shown in Figure 4, and may be 'in-ear' type headphones that can be inserted into the wearer's ears. Of course, any suitable type of headphones may be used in conjunction with the present invention.

Referring again to Figure 1, the computer 114 also comprises a control interface 134 to control the operation of the device 100 via voice-activation. Hence, the wearer can issue spoken commands to the device 100 in order to initiate or inhibit some particular function. The control interface 134 comprises a miniature type microphone 136 that is operable to receive the spoken commands. The microphone 136 is located on the left-hand arm 106 of the frame 104, as best shown in Figure 3 and 4. Of course, the microphone 136 could be located anywhere on the frame 104, or else about the body of the wearer, in order to achieve the same function.

The wearer is able to control any operation of the optical device via the control interface 134, including switching the device ON or OFF; instructing the object identification algorithm to ignore certain objects or object types; to switch the speech synthesiser ON or OFF (to commence or inhibit the output of spoken words recognised in the images); and to commence or terminate recording of a sequence of images (for later processing).

As mentioned earlier, the computer 114 also comprises an adaptive learning means 128 that is operable to learn different objects so as to discriminate between different object types. In addition, the adaptive learning means 128 is also configured to learn new text (e.g. words, phrases etc.) based on the textual content in the captured video images.

The adaptive learning means 128 is implemented in software and can have different modes of learning that allow it to save new objects into the database 126. One mode is initiated by the wearer, such that objects are presented to the optical device 100 and the wearer then instructs the device to 'learn' the new object. The wearer initiates the learning by issuing the spoken command "LEARN" to the control interface 134 (via microphone 136), which triggers the adaptive learning means 128 to record a video sequence via the cameras 112. The recorded video sequence is then analysed to build up an object recognition file for that new object, and depending on the particular implementation can also assign a category to that object.

The analysis of the recorded video sequence is performed 'OFFLINE' (e.g. while the optical device 100 is not in active use by the wearer) and remotely from the optical device 100. In some examples, the recorded video sequences are uploaded to a remote secure server, as maintained by the equipment manufacturer or developer etc., but may alternatively also be analysed locally by the wearer's personal computer (e.g. desktop or laptop etc.). The need for a 'secure' server is to allay any concerns of the wearer regarding the uploading of their personal video sequences. Therefore, video files can also be encrypted in some examples to prevent unauthorised viewing of the sequences, and would in any event be automatically deleted from the server after analysis had been completed.

Carrying out the analysis remotely to the device reduces processing overheads on the processer 116 of the computer 114, which otherwise could diminish performance of the optical device 100 during use, or else shorten battery life etc. In either case, bespoke software performs the object recognition and generates an object recognition file for subsequent download to the database 126 of the computer 114. In this way, new objects can be added to the database 126 over time, thereby building up a customised collection of object recognition files for the wearer.

It is also possible for the processing of the video sequence to be carried out gradually during use of the device 100, by making use of any spare processing cycles of the processor 116 or by exploiting any 'idle time' when the device and/or software is not currently carrying out an operation etc. Alternatively, the processing can also be performed when the device 100 is not in use and is recharging etc.

Another learning mode, which may or may not be invoked in some examples, is a behavioural led form of learning, such that the behaviour of the wearer is monitored and deduced in order to update the database 126. An orientation determining means, in the form of a tri-axial gyroscope 138 (see Figures 1 & 2) is used to perform an approximate estimate of the wearer's ongoing behaviour. For example, if the device 100 is functioning and the gyroscope 138 indicates that the wearer is stationary, then it is reasonable to assume that the wearer is engaged in a meaningful task. If the object recognition algorithms do not recognise any objects or text in the captured images, the adaptive learning means 128 can be set to automatically begin recording a video sequence for subsequent object recognition (either offline and/or remotely etc.). Thus, any objects associated with that meaningful task that are not yet in the database 126 can be analysed and appropriate object recognition files can be generated and saved for use in future objection identification.

The tri-axial gyroscope may be a microchip packaged MEMS gyroscope. However, a tri-axial accelerometer may alternatively be used.

Referring again to Figure 1, optical device 100 is powered by an internal battery 140, which is rechargeable. The battery 140 provides electrical power to the computer 114, together with the LED arrays 102 and the cameras 112 via a wired electrical connection (not shown for clarity). Of course, any suitable battery or battery pack may be used in order to provide power to the optical device 100 of the present invention, provided that the portability and/or wearability of the device is not unduly hindered.

It is to be understood that implementation of any of the algorithmic routines for image processing, object identification, facial recognition, optical character recognition, Text-to-Speech and voice-activated control etc. can be achieved via any programming language and may make use of any standard or bespoke libraries and source codes etc. Hence, in some examples the software may be implemented via the National Instruments Lab VIEW development environment (http://www.ni.com/labview/); while in other examples all APIs and algorithms may be written in C/C + +.

The processor 116 of computer 114 is ideally a CPU designed for mobile computing applications, and as such has a relatively small form factor and more efficient power consumption compared to other chip designs. Hence, the computer 114 may be implemented on a ARM platform, which utilises RISC architecture, for example, a dual-core ARM Cortex-A9 processor. For ARM platform implementations, the algorithmic routines may be programmed in C++ and the open source code OpenCV (http://opencv.willowgarage.com/wiki/) may be used for image processing.

The open source libraries provided by Carnegie Mellon University may be used to provide the necessary speech and voice recognition functionality. Hence, a suitable speech synthesis library for use with the optical device of the present invention is Flite (http://www.speech.cs.cmu.edu/flite/), while voice recognition can be achieved via library CMUSphinx (http://cmusphinx.sourceforge.net/). Text recognition may be achieved via the open source code Tesseract (http://code.google.com/p/tesseractocr/) or OCRopus (http://code.google.com/p/ocropus/).

The LED arrays may be controlled via the SPI communication protocol or any other serial protocol, for example I²C or UART etc.

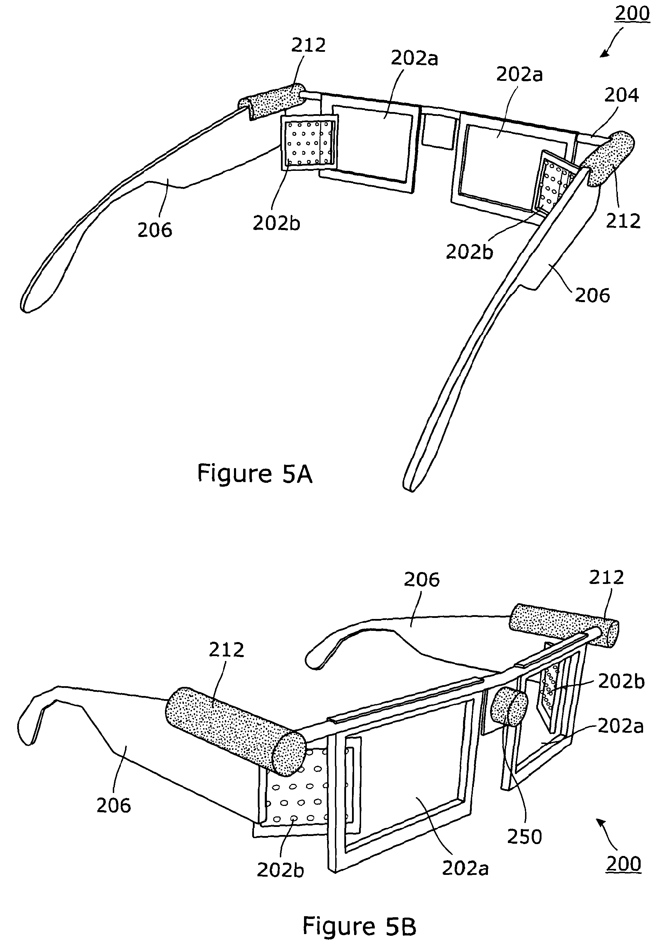

Referring now to Figures 5A & 5B, there is shown an optical device according to another preferred embodiment of the present invention. In this embodiment, the optical device 200 comprises a compound display, which includes first and second arrays 202a, 202b of a plurality of addressable light-sources. The compound display is mounted to, or is otherwise integrated with, a support frame, which in the example of Figures 5A & 5B is a spectacle frame 204 having side arms 206, similar to the frame 104 of the earlier embodiments described above.

The compound display is arranged to provide an optical stimulus to the wearer's central and/or peripheral vision by way of the first and second arrays (202a, 202b). By 'central vision' we mean the wearer's vision substantially along his/hers line of sight (typically looking forward or ahead), while 'peripheral vision' is intended to encompass any lateral or side of the eye visual function, and typically relates to the wearer's vision at an angle to their direct line of sight.

As shown in Figure 5A, the first array 202a is different to that of the second array 202b, and comprises a greater number of addressable light-sources than the second array. The first array 202a is a transparent OLED (organic light-emitting diode) 2D display comprising individually addressable LEDs. The second array 202b is a scaled down version of the spaced LED arrays as described in relation to the earlier embodiments, and is disposed adjacent to a respective one of the arms 206 of the spectacle frame 204, with the array being angled to the OLED array to permit the wearer's peripheral vision to be optically stimulated by selectively driving one or more of the spaced LEDs. The second array 202b is also transparent.

Hence, in this example, the wearer's central vision may be stimulated by the higher resolution OLED display 202a, while their peripheral vision may be stimulated by the lower resolution spaced LED array 202b. This arrangement has significant advantages, not least, in terms of the increased informational content that can be conveyed to the wearer, by way of the combined use of two separate displays for each respective eye.

Moreover, it has been found that during testing some visually impaired wearers retained sufficient visual resolution to be able to discern the spacing between the light sources in the embodiments of Figures 1 to 4. Therefore, for such individuals, the higher resolution display may be more beneficial, as they are able to discern greater detail as compared to more severely afflicted visually impaired wearers.

The frame 204 also supports a pair of stereoscopic cameras 212, as described in relation to the earlier embodiments. The cameras 212 and software are operable to generate a depth map of the wearer's immediate environment, as discussed earlier. Therefore, the software acquires video data from the two cameras 212, which are fixed and separated by a known distance, and then compares the positions of a large number of features common to both cameras, in order to calculate the distance to located objects within the scene. The image is then converted into a depth map, with nearer objects appearing brighter, while objects further away fade to black. As a result, the present device provides an intuitive real-time display that presents the relative sizes and distances to objects within the wearer's immediate environment.

Referring again to Figure 5B, the device 200 also comprises an ultrasonic range finder 250, which is mounted on the bridge of the frame 204. The principal function of the range finder is to detect objects less than about 1 metre away from the wearer and to provide a substantially 'fail-safe' mechanism to avoid collisions with objects that are undetectable by the pair of cameras 212, for example, glass doors etc. Information gathered from the ultrasonic range finder 250 is conveyed to the wearer using the arrays 202a, 202b, in accordance with the generated depth image or map. Hence, for example, the central portion of the arrays become brighter as objects approach the wearer (or as the wearer approaches the objects) and vice versa.

Although the optical device and method of the present invention are ideally suited for visually-impaired individuals who retain at least some light and/or colour discrimination, it will be recognised that one or more of the principles of the invention may extend to other visual aid or augmented reality applications, whereby the visual impairment may not be especially significant or relevant but assisted-viewing may be desirable as a teaching or training aid for mobility sufferers or where an individual has learning difficulties etc. In particular, it is envisaged that the present invention could also be useful for dementia sufferers who could benefit from a device that improves their ability to recognise faces and locations etc.

The above embodiments are described by way of example only. Many variations are possible without departing from the invention.

## Claims

1. An optical device (100) for a visually-impaired individual, comprising:
a first regular matrix (102) of individual light sources that are spaced from one another by a predetermined amount;
a support (104) arranged to maintain the first regular matrix (102) in proximate relation to at least one eye of the individual; and
a camera-based image capture device comprising an optical lens configured to capture images, via the lens, of at least part of the individual's immediate environment;
wherein the first regular matrix (102) is configured to convey information to the individual by selectively illuminating one or more of the individual light sources based on the content of the captured images.

2. The optical device (100) of Claim 1, wherein the support (104) is arranged to maintain the regular matrix (102) at a distance from the eye substantially closer than the minimum focal length of the eye.

3. The optical device (100) of Claim 1 or Claim 2, wherein the regular matrix (102) is a matrix (102) of light emitting diodes, and optionally,
wherein the light emitting diodes are individually addressable.

4. The optical device (100) of any preceding claim, further comprising a second regular matrix (102) of individual light sources that are spaced from one another by a predetermined amount, each regular matrix (102) configured to convey information to a respective eye of the individual, or
the optical device (100) of any preceding claim, wherein the support (104) comprises a spectacle frame (104), and optionally, wherein each regular matrix (102) is integrated into a respective display in the shape of a spectacle lens fitted to the frame (104).

5. The optical device (100) of any preceding claim, wherein the camera-based image capture device is mounted on the support (104), and optionally,
wherein the camera-based image capture device comprises at least one wide-angle camera, and optionally,
wherein the wide-angle camera is a video camera (112).

6. The optical device (100) of any preceding claim, further comprising a computing device (114) for controlling the regular matrix (102) and/or the image capture device, and optionally,
wherein the computing device (114) is adapted to be separately wearable by the individual, and optionally,
wherein the computing device (114) comprises an image processing means (124) operable to identify objects in the captured images, and optionally,
wherein the identification involves determining one or more of object type, spatial size, position relative to the individual and distance to the object.

7. The optical device (100) of Claim 6, wherein the image processing means (124) is further operable to perform text recognition based on textual content in the captured images, and optionally,
further comprising a speech synthesiser (130) operable to provide a spoken output corresponding to the recognised text.

8. The optical device (100) of any preceding claim, further comprising an audio output device, and optionally,
wherein the audio output device comprises a pair of headphones (132), or the optical device (100) of any preceding claim, further comprising a control interface (134) to control the operation of the device (100), and optionally,
wherein the control interface (134) is voice activated, and optionally,
wherein the control interface (134) comprises at least one microphone (136) operable to receive spoken commands.

9. The optical device (100) of Claim 6, wherein the computing device (114) further comprises an adaptive learning means (128) operable to learn new objects and/or text based on the content of the captured images, and optionally,
wherein the adaptive learning means (128) is configured to learn while the device (100) is not active, and optionally,
wherein the adaptive learning means (128) is configured to activate its learning mode in response to a spoken command.

10. The optical device (100) of any preceding claim, further comprising an orientation determining means to determine the orientation of the support (104) relative to the individual's immediate environment, and optionally, wherein the orientation determining means comprises one of a gyroscope (138) or an accelerometer, or
the optical device of any preceding claim, further comprising a power supply (140).

11. The optical device (200) of Claim 1, further comprising:
a compound display comprising the first regular matrix (202a) and a second regular matrix (202b) each of a plurality of individual addressable light-sources;
wherein the support (204) is arranged to maintain the matrices (202a,202b) in proximate relation to at least one eye of the individual, such that the second regular matrix (220b) is angled relative to the first regular matrix (220a);
and
wherein the first and second regular matrices (202a,202b) are configured to provide an optical stimulus to the individual's central and/or peripheral vision by selectively illuminating one or more of the addressable light sources based on the content of the captured images to thereby convey information to the individual.

12. The optical device (200) of Claim 11, wherein the first regular matrix (220a) is different to the second regular matrix (220b), and optionally, wherein the first regular matrix (220a) comprises a greater number of addressable light-sources than the second regular matrix (220b), or the optical device of Claim 11, wherein the first regular matrix (220a) is of a higher resolution than the second regular matrix (220b), or
the optical device of Claim 11, wherein the first regular matrix (220a) is an OLED display.

13. The optical device (200) of Claim 11 or Claim 12, wherein the second regular matrix (220b) is configured to provide an optical stimulus to the individual's peripheral vision only, or
the optical device of Claim 11 or Claim 12, wherein the compound display further comprises third and fourth regular matrices of individual addressable light-sources, the first and second regular matrices (202a,202b) and the third and fourth regular matrices being configured to convey information to a respective eye of the individual.

14. A computer program product, comprising program code means being adapted to operate the device of any of Claims 1 to 13 when the program code is executed on a computing device comprised in the device of any of Claims 1 to 13, and causing said computing device to execute the following steps:
- capturing images, via the camera-based image capture device of at least a part of the individual's immediate environment;
- processing the images to identify the content of the captured images;
and displaying information based on the content of the captured images on the first regular matrix (102) of discrete light sources by selectively illuminating one or more of the discrete light sources to generate a predetermined pattern of illumination indicative of the said information.

## Patentansprüche

1. Eine optische Vorrichtung (100) für einen Sehbehinderten, die Folgendes aufweist:
ein erstes regelmäßiges Rastermuster (102) einzelner Lichtquellen, die in einem bestimmten Abstand voneinander getrennt angebracht sind;
Eine Fassung (104), die so angeordnet ist, dass sie das erste regelmäßige Rastermuster (102) in die Nähe mindestens eines Auges des Individuums bringen kann; und
eine kamerabasierte Bilderfassungsvorrichtung, die eine optische Linse aufweist, die so gestaltet ist, dass sie Bilder, über die Linse, mindestens eines Teils der unmittelbaren Umgebung des Individuums erfassen kann;
wobei das erste regelmäßige Rastermuster (102) so gestaltet ist, dass es die Informationen an das Individuum übermittelt, durch selektives Beleuchten einer oder mehrerer einzelner Lichtquellen, auf der Basis des Contents der erfassten Bilder.

2. Die optische Vorrichtung (100) gemäß Anspruch 1, wobei die Fassung (104) so angeordnet ist, dass sie das regelmäßige Rastermuster (102) in einem Abstand vom Auge hält, der im Wesentlichen geringer ist als die Mindestbrennweite des Auges.

3. Die optische Vorrichtung (100) gemäß Anspruch 1 oder Anspruch 2, wobei das regelmäßige Rastermuster (102) ein Rastermuster (102) lichtemittierender Dioden ist und optional,
wobei die lichtemittierenden Dioden individuell aufrufbar sind.

4. Die optische Vorrichtung (100) eines der vorhergehenden Ansprüche, die darüberhinaus ein zweites regelmäßiges Rastermuster (102) der einzelnen Lichtquellen aufweist, die in einem vorbestimmten Abstand voneinander getrennt angebracht sind, jedes regelmäßige Rastermuster (102) ist so gestaltet, dass es die Informationen an ein Auge des Individuums überträgt, oder
die optische Vorrichtung (100) eines der vorhergehenden Ansprüche, wobei die Fassung (104) ein Brillengestell (104) aufweist, und optional, wobei
jedes regelmäßige Rastermuster (102) in ein entsprechendes Display in Form einer Brillenlinse, die in die Fassung (104) eingesetzt ist, integriert ist.

5. Die optische Vorrichtung (100) eines der vorhergehenden Ansprüche, wobei die kamerabasierte Bilderfassungsvorrichtung auf der Fassung (104) montiert ist, und optional,
wobei die kamerabasierte Bilderfassungsvorrichtung mindestens eine Weitwinkelkamera aufweist, und optional,
wobei die Weitwinkelkamera eine Videokamera (112) ist.

6. Die optische Vorrichtung (100) eines der vorhergehenden Ansprüche, die darüberhinaus ein Computersystem (114) aufweist, für die Steuerung des regelmäßigen Rastermusters (102) und/oder die Bilderfassungsvorrichtung, und optional,
wobei das Computersystem (114) so angepasst ist, dass es vom Individuum getrennt getragen werden kann, und optional,
wobei das Computersystem (114) eine Bildverarbeitungsvorrichtung (124) aufweist, die Objekte in den erfassten Bildern identifizieren kann, und optional,
wobei die Identifikation die Bestimmung eines oder mehrerer Objekttypen, Raumgrößen, Positionen bezüglich des Individuums und Abstände zum Objekt beinhaltet.

7. Die optische Vorrichtung (100) gemäß Anspruch 6, wobei die Bildverarbeitungsvorrichtung (124) darüberhinaus die Texterkennung auf der Basis des Textinhalts in den erfassten Bildern ausführt, und optional,
darüberhinaus einen Sprachgenerator (130) aufweist, der eine dem erkannten Text entsprechende Sprachausgabe bereitstellt.

8. Die optische Vorrichtung (100) eines der vorhergehenden Ansprüche, die darüberhinaus eine Audioausgabevorrichtung aufweist, und optional,
wobei die Audioausgabevorrichtung ein Paar Kopfhörer (132) oder die optische Vorrichtung (100) eines der vorhergehenden Ansprüche aufweist, und darüberhinaus eine Bedieneroberfläche (134) aufweist, um den Vorgang der Vorrichtung (100) zu steuern, und optional,
wobei die Bedieneroberfläche (134) stimmaktiviert ist, und optional,
wobei die Bedieneroberfläche (134) mindestens ein Mikrofon (136) aufweist, das die Sprachbefehle empfangen kann.

9. Die optische Vorrichtung (100) gemäß Anspruch 6, wobei das Computersystem (114) darüberhinaus eine adaptive Lernvorrichtung (128) aufweist, die neue Objekte und/oder Texte auf der Basis des Content der erfassten Bilder erlernen kann, und optional,
wobei die adaptive Lernvorrichtung (128) so gestaltet ist, dass sie lernt, während das Gerät (100) nicht aktiv ist, und optional,
wobei die adaptive Lernvorrichtung (128) so gestaltet ist, dass sie ihren Lernmodus als Reaktion auf einen gesprochenen Befehl aktiviert.

10. Die optische Vorrichtung (100) eines der vorhergehenden Ansprüche, die darüberhinaus eine Ausrichtungsfestlegungsvorrichtung aufweist, um die Ausrichtung der Fassung (104) im Verhältnis zur unmittelbaren Umgebung des Individuums festlegt und optional, wobei die Ausrichtungsfestlegungsvorrichtung entweder ein Gyroskop (138) oder einen Beschleunigungsmesser aufweist, oder
die optische Vorrichtung eines der vorhergehenden Ansprüche, die darüberhinaus eine Stromzufuhr (140) aufweist.

11. Die optische Vorrichtung (200) gemäß Anspruch 1, die darüberhinaus Folgendes aufweist:
ein Verbindungs-Display, das das erste regelmäßige Rastermuster (202a) und ein zweites regelmäßiges Rastermuster (202b) einer Vielzahl von einzeln aufrufbarer Lichtquellen aufweist;
wobei die Fassung (204) so angeordnet ist, dass sie die Rastermuster (202a,202b) in die Nähe mindestens eines Auges des Individuums bringt, so dass das zweite regelmäßige Rastermuster (220b) im Verhältnis zum ersten regelmäßigen Rastermuster (220a) abgewinkelt ist; und
wobei das erste und zweite regelmäßige Rastermuster (202a,202b) so gestaltet sind, dass sie dem zentralen und/oder peripheren Sehen des Individuums einen optischen Stimulus bieten, durch selektive Beleuchtung einer oder mehrerer aufrufbarer Lichtquellen, auf der Basis des Content der erfassten Bilder, um dadurch die Informationen an das Individuum zu übermitteln.

12. Die optische Vorrichtung (200) gemäß Anspruch 11, wobei sich das erste regelmäßige Rastermuster (220a) vom zweiten regelmäßigen Rastermuster (220b) unterscheidet, und optional, wobei das erste regelmäßige Rastermuster (220a) eine größere Zahl von aufrufbaren Lichtquellen aufweist als das zweite regelmäßige Rastermuster (220b) oder die optische Vorrichtung gemäß Anspruch 11, wobei das erste regelmäßige Rastermuster (220a) eine höhere Auflösung hat als das zweite regelmäßige Rastermuster (220b), oder
die optische Vorrichtung gemäß Anspruch 11, wobei das erste regelmäßige Rastermuster (220a) ein OLED-Display ist.

13. Die optische Vorrichtung (200) gemäß Anspruch 11 oder Anspruch 12, wobei das zweite regelmäßige Rastermuster (220b) so gestaltet ist, dass es einen optischen Stimulus nur für das periphere Sehen des Individuums bietet, oder
die optische Vorrichtung gemäß Anspruch 11 oder Anspruch 12, wobei das Verbindungs-Display darüberhinaus dritte und vierte regelmäßige Rastermuster einzelner aufrufbarer Lichtquellen aufweist, das erste und zweite regelmäßige Rastermuster (202a,202b) und das dritte und vierte regelmäßige Rastermuster sind dabei so gestaltet, dass sie die Informationen an ein entsprechendes Auge des Individuums übertragen.

14. Ein Computer-Programm-Produkt, das eine Programm-Code-Vorrichtung aufweist, die so angepasst ist, dass sie die Vorrichtung gemäß eines der Ansprüche 1 bis 13 betreibt, wenn der Programm-Code in einem Computersystem ausgeführt wird, das in der Vorrichtung gemäß eines der Ansprüche 1 bis 13 enthalten ist, und das das Computersystem verwendet, um die folgenden Schritte auszuführen:
- das Erfassen von Bildern über die kamerabasierte Bilderfassungsvorrichtung von mindestens einem Teil der unmittelbaren Umgebung des Individuums;
- das Verarbeiten der Bilder, um den Content der erfassten Bilder zu identifizieren;
und die Anzeige der Informationen auf der Basis des Content der erfassten Bilder auf dem ersten regelmäßigen Rastermuster (102) der einzelnen Lichtquellen durch selektive Beleuchtung einer oder mehrerer einzelner Lichtquellen, um ein vorbestimmtes Beleuchtungsmuster zu erzeugen, das die besagten Informationen anzeigt.

## Revendications

1. Un dispositif optique (100) destiné à une personne malvoyante, comprenant :
une première matrice régulière (102) de sources lumineuses individuelles qui sont espacées les unes des autres par une quantité prédéterminée,
un support (104) agencé de façon à maintenir la première matrice régulière (102) en relation de proximité avec au moins un oeil de la personne, et
un dispositif de capture d'images de type caméra comprenant une lentille optique configurée de façon à capturer des images, par l'intermédiaire de la lentille, d'au moins une partie de l'environnement immédiat de la personne,
où la première matrice régulière (102) est configurée de façon à acheminer des informations à la personne par l'éclairage sélectif d'une ou de plusieurs des sources lumineuses individuelles en fonction du contenu des images capturées.

2. Le dispositif optique (100) selon la Revendication 1, où le support (104) est agencé de façon à maintenir la matrice régulière (102) à une distance de l'oeil sensiblement plus proche que la longueur focale minimale de l'oeil.

3. Le dispositif optique (100) selon la Revendication 1 ou 2, où la matrice régulière (102) est une matrice (102) de diodes électroluminescentes, et éventuellement,
où les diodes électroluminescentes sont adressables individuellement.

4. Le dispositif optique (100) selon l'une quelconque des Revendications précédentes, comprenant en outre une deuxième matrice régulière (102) de sources lumineuses individuelles qui sont espacées les unes des autres par une quantité prédéterminée, chaque matrice régulière (102) étant configurée de façon à acheminer des informations à un oeil respectif de la personne, ou
le dispositif optique (100) selon l'une quelconque des Revendications précédentes, où le support (104) comprend une monture de lunettes (104), et éventuellement, où
chaque matrice régulière (102) est intégrée à un écran respectif de la forme d'une lentille de lunettes fixée à la monture (104).

5. Le dispositif optique (100) selon l'une quelconque des Revendications précédentes, où le dispositif de capture d'images de type caméra est monté sur le support (104), et éventuellement,
où le dispositif de capture d'images de type caméra comprend au moins une caméra à grand angulaire, et éventuellement,
où la caméra à grand angulaire est une caméra vidéo (112).

6. Le dispositif optique (100) selon l'une quelconque des Revendications précédentes, comprenant en outre un dispositif informatique (114) destiné à la commande de la matrice régulière (102) et/ou du dispositif de capture d'images, et éventuellement,
où le dispositif informatique (114) est adapté de façon à pouvoir être porté séparément par la personne, et éventuellement,
où le dispositif informatique (114) comprend un moyen de traitement d'images (124) conçu de façon à identifier des objets dans les images capturées, et éventuellement,
où l'identification implique la détermination d'un ou de plusieurs éléments parmi type d'objet, taille spatiale, position par rapport à la personne et distance à l'objet.

7. Le dispositif optique (100) selon la Revendication 6, où le moyen de traitement d'images (124) est conçu en outre de façon à exécuter une reconnaissance textuelle en fonction d'un contenu textuel dans les images capturées, et éventuellement,
comprenant en outre un synthétiseur de la parole (130) conçu de façon à fournir une sortie vocale correspondant au texte reconnu.

8. Le dispositif optique (100) selon l'une quelconque des Revendications précédentes, comprenant en outre un dispositif de sortie audio, et éventuellement,
où le dispositif de sortie audio comprend une paire d'écouteurs (132), ou le dispositif optique (100) selon l'une quelconque des Revendications précédentes comprenant en outre une interface de commande (134) destinée à la commande du fonctionnement du dispositif (100), et éventuellement,
où l'interface de commande (134) est activée vocalement, et éventuellement,
où l'interface de commande (134) comprend au moins un microphone (136) conçu de façon à recevoir des commandes vocales.

9. Le dispositif optique (100) selon la Revendication 6, où le dispositif informatique (114) comprend en outre un moyen d'apprentissage adaptatif (128) conçu de façon à apprendre de nouveaux objets et/ou textes en fonction du contenu des images capturées, et éventuellement,
où le moyen d'apprentissage adaptatif (128) est configuré de façon à apprendre pendant que le dispositif (100) n'est pas actif, et éventuellement,
où le moyen d'apprentissage adaptatif (128) est configuré de façon à activer son mode d'apprentissage en réponse à une commande vocale.

10. Le dispositif optique (100) selon l'une quelconque des Revendications précédentes, comprenant en outre un moyen de détermination d'orientation destiné à déterminer l'orientation du support (104) par rapport à l'environnement immédiat de la personne, et éventuellement, où le moyen de détermination d'orientation comprend un élément parmi un gyroscope (138) ou un accéléromètre, ou
le dispositif optique selon l'une quelconque des Revendications précédentes comprenant en outre une alimentation électrique (140).

11. Le dispositif optique (200) selon la Revendication 1, comprenant en outre :
un écran composite comprenant la première matrice régulière (202a) et une deuxième matrice régulière (202b), chaque source de lumière d'une pluralité de sources de lumière adressables individuelles,
où le support (204) est agencé de façon à maintenir les matrices (202a, 202b) en relation de proximité avec au moins un oeil de la personne, de sorte que la deuxième matrice régulière (220b) soit inclinée par rapport à la première matrice régulière (220a),
et
où les première et deuxième matrices régulières (202a, 202b) sont configurées de façon à fournir un stimulus optique à la vision périphérique et/ou centrale de la personne par l'éclairage sélectif d'une ou de plusieurs des sources lumineuses adressables en fonction du contenu des images capturées, de façon à acheminer ainsi des informations à la personne.

12. Le dispositif optique (200) selon la Revendication 11, où la première matrice régulière (220a) est différente de la deuxième matrice régulière (220b), et éventuellement, où la première matrice régulière (220a) comprend un nombre plus élevé de sources de lumière adressables que la deuxième matrice régulière (220b), ou le dispositif optique selon la Revendication 11, où la première matrice régulière (220a) présente une résolution plus élevée que la deuxième matrice régulière (220b), ou
le dispositif optique selon la Revendication 11, où la première matrice régulière (220a) est un écran OLED.

13. Le dispositif optique (200) selon la Revendication 11 ou 12, où la deuxième matrice régulière (220b) est configurée de façon à fournir un stimulus optique à la vision périphérique de la personne uniquement, ou
le dispositif optique selon la Revendication 11 ou 12, où l'écran composite comprend en outre une troisième et une quatrième matrices régulières de sources de lumière adressables individuelles, les première et deuxième matrices régulières (202a,202b) et les troisième et quatrième matrices régulières étant configurées de façon à acheminer des informations à un oeil respectif de la personne.

14. Un produit de programme informatique, comprenant un moyen de code de programme qui est adapté de façon à actionner le dispositif selon l'une quelconque des Revendications 1 à 13 lorsque le code de programme est exécuté sur un dispositif informatique contenu dans le dispositif selon l'une quelconque des Revendications 1 à 13, et à amener ledit dispositif informatique à exécuter les opérations suivantes :
- la capture d'images, par l'intermédiaire du dispositif de capture d'images de type caméra, d'au moins une partie de l'environnement immédiat de la personne,
- le traitement des images de façon à identifier le contenu des images capturées,
et l'affichage d'informations en fonction du contenu des images capturées sur la première matrice régulière (102) de sources lumineuses discrètes par l'éclairage sélectif d'une ou de plusieurs des sources lumineuses discrètes de façon à générer un modèle prédéterminé d'éclairage indicatif desdites informations.
